# EUROPEAN PATENT APPLICATION

(11) **EP 3 995 504 A1**
(43) Date of publication of application: **11.05.2022**
(21) Application number: 20834280.8
(22) Date of filing: 24.06.2020
(51) Int. Cl.: C07K 1/14, C12N 7/02

(54) **METHOD FOR PURIFYING VIRUS OR VIRUS-LIKE PARTICLE**

(30) Priority: 04.07.2019 JP 2019125243
(71) Applicant: KANEKA CORPORATION, Osaka 530-8288 (JP)
(72) Inventor: NISHIHACHIJYO, Masakatsu, Takasago-shi, Hyogo 676-8688 (JP); SUEOKA, Takuma, Takasago-shi, Hyogo 676-8688 (JP); YAURA, Hisako, Takasago-shi, Hyogo 676-8688 (JP)
(74) Representative: Hoffmann Eitle
(86) International application number: PCT/JP2020/024804
(87) International publication number: WO 2021/002257

(57) **Abstract**

The objective of the present invention is to provide a method capable of purifying a virus or a virus-like particle easily. The method for purifying a virus or a virus-like particle according to the present invention is characterized in comprising the step of contacting a liquid comprising the virus or the virus-like particle with a water-insoluble inorganic compound, wherein the water-insoluble inorganic compound comprises one or more elements selected from magnesium, calcium and aluminum.

## Description

### TECHNICAL FIELD

The present invention relates to a method capable of purifying a virus or a virus-like particle easily.

### BACKGROUND ART

In general, a genome is purified using a commercially available kit from a sample and a base sequence of the genome is determined in order to identify a virus. The data has been established as to what virus has what genome sequence, and the genus of a virus can be identified by determining the genome sequence. It is necessary to purify the virus with high purity for that purpose. In addition, a virus is used as a vector for introducing a specific gene into a cell in gene therapy, and the virus used in gene therapy must be of course highly purified. A virus without toxicity and an inactive virus-like particle are used as vaccine, and must be purified to a high degree of purity.

A virus and a virus-like particle are produced by a transformed cell, a hen egg or the like, and purified from a disrupted cell suspension and a chorioallantoic fluid. Such disrupted cell suspension and chorioallantoic fluid contain many impurities. For example, a virus and a virus-like particle are purified by ultracentrifugation, membrane separation and chromatography. An ultracentrifugation method, however, requires a dedicated device and is difficult to be carried out in large scale. Membrane separation and chromatography require an expensive material, much trouble and a long time to set a condition for high purification. An easier purification method is, therefore, developed in place of the above-described purification methods or in order to be preliminarily conducted before the above-described purification methods are performed.

For example, Patent document 1 discloses a method for purifying a virus by adjusting a concentration of an inorganic salt and pH of a sample liquid containing the virus in order to easily adsorb humic acid, which is a mixture of high molecular organic acids synthesized chemically/biologically from degradant derived from a residue of a plant and remains of a microorganism or plankton, on the surface of hydrophobic beads. A disrupted cell suspension and a chorioallantoic fluid which contain a virus also contain an impurity such as a protein and a nucleic acid; therefore, it is unclear whether or not a virus can be purified from such protein and nucleic acid by the above-described method.

Patent document 2 discloses a method for capturing a virus-like particle to be purified on an extension bed of an adsorbent. An insoluble inorganic compound such as magnesium oxide is exemplified as a material for an adsorbent. Such an insoluble inorganic compound is merely exemplified as an inactive core material for an adsorbent, and the adsorbent which is actually used in the experiment is an adsorbent having an ion exchanger such as diethylaminoethyl (DEAE) on the surface as a ligand. It may be therefore needed to adjust a condition such as a salt concentration and pH of each sample containing a virus-like particle in order to selectively adsorb the target virus-like particle and not to adsorb the other protein and nucleic acid on an adsorbent.

### PRIOR ART DOCUMENT

### PATENT DOCUMENT

Patent document 1: WO 2015/111606
Patent document 2: JP 2017-55766 A

### DISCLOSURE OF THE INVENTION

### PROBLEMS TO BE SOLVED BY THE INVENTION

A method for easily purifying a virus or a virus-like particle from an impurity such as the other protein and nucleic acid has not been established as described above.

Accordingly, the objective of the present invention is to provide a method capable of purifying a virus or a virus-like particle easily.

### MEANS FOR SOLVING THE PROBLEMS

The inventors of the present invention repeated intensive studies in order to solve the above-described problems. As a result, the inventors completed the present invention by finding that a virus and a virus-like particle can be easily purified at a low cost, since a virus and a virus-like particle are hardly adsorbed on the specific water-insoluble inorganic compound but the other protein and nucleic acid are selectively and easily adsorbed among the components contained in a disrupted cell suspension containing the virus or the virus-like particle.

The present invention is hereinafter described.

[1] A method for purifying a virus or a virus-like particle, comprising the step of contacting a liquid comprising the virus or the virus-like particle with a water-insoluble inorganic compound,
   wherein the water-insoluble inorganic compound comprises one or more elements selected from magnesium, calcium and aluminum.
[2] A method for producing a virus or a virus-like particle, comprising the step of purifying the virus or the virus-like particle by contacting a liquid comprising the virus or the virus-like particle with a water-insoluble inorganic compound,
   wherein the water-insoluble inorganic compound comprises one or more elements selected from magnesium, calcium and aluminum.
[3] The method according to the above [1] or [2], wherein the water-insoluble inorganic compound is one or more selected from magnesium carbonate, magnesium hydroxide, magnesium oxide, calcium sulfate and aluminum oxide.
[4] The method according to any one of the above [1] to [3], further comprising the step of contacting the liquid comprising the virus or the virus-like particle with an activated carbon.
[5] The method according to any one of the above [1] to [4], further comprising the step of purifying the virus or the virus-like particle by chromatography.
[6] The method according to the above [5], wherein the chromatography is affinity chromatography.
[7] The method according to any one of the above [1] to [6], wherein the virus or the virus-like particle is an adeno-associated virus or a virus-like particle derived from an adeno-associated virus.
[8] The method according to any one of the above [1] to [7], wherein a protein and/or a nucleic acid other than the virus or the virus-like particle is adsorbed on the water-insoluble inorganic compound.
[9] The method according to any one of the above [1] to [8], wherein the liquid is an animal cell culture fluid or a processed material thereof.

### EFFECT OF THE INVENTION

The adsorbent used in the present invention method is very inexpensive and does not require the time and effort needed by an adsorbent having a ligand on the surface, since the present invention adsorbent is the specific water-insoluble inorganic compound to which an ion-exchange group and a ligand are not needed to be bound. In addition, a virus and a virus-like particle can be purified by the adsorbent used in the present invention method, since the total amount of a protein and a nucleic acid contained in a disrupted cell suspension containing a virus or a virus-like particle can be reduced by the adsorbent used in the present invention method but the adsorbent used in the present invention method has low affinity for a virus and a virus-like particle. The present invention is, therefore, industrially very useful as a technology to identify a virus, and easily and efficiently purify a virus or a virus-like particle needed for gene therapy, vaccine therapy or the like.

### BRIEF DESCRIPTION OF THE DRAWINGS

Figure 1 is a graph to show the quantitative value results of total proteins and adeno-associated virus in the case where the adeno-associated virus was purified from a disrupted cell liquid using water-insoluble or water-soluble inorganic compound.
Figure 2 is a graph to show the quantitative value results of total proteins and adeno-associated virus in the case where the adeno-associated virus was purified from a disrupted cell liquid using basic magnesium carbonate as a water-insoluble inorganic compound and the concentration thereof was variously changed.
Figure 3 is a graph to show the quantitative value results of total proteins and adeno-associated virus in the case where the adeno-associated virus was purified using basic magnesium carbonate as a water-insoluble inorganic compound from disrupted cell liquids having various salt concentrations.
Figure 4 is a graph to show the quantitative value results of total proteins and adeno-associated virus in the case where the adeno-associated virus was purified from a disrupted cell liquid using a water-insoluble inorganic compound with or without an activated carbon.
Figure 5 is a graph to show the quantitative value results of total proteins, adeno-associated virus and DNA in the case where the adeno-associated virus was purified using basic magnesium carbonate as a water-insoluble inorganic compound from a disrupted cell liquid treated by an endonuclease.
Figure 6 is a particle size distribution of light basic magnesium carbonate used in the Example described later.
Figure 7 is a graph to show the amounts of adeno-associated virus, protein and DNA in the case where a nucleolytic treatment liquid by an endonuclease was further treated or not treated using basic magnesium carbonate of the present invention.
Figure 8 is a graph to show the relation between an amount of basic magnesium carbonate and a protein removal rate in the case where a nucleolytic treatment liquid by an endonuclease was further treated using basic magnesium carbonate according to the present invention.
Figure 9 are graphs to show particle size distributions of a purified AAV liquid, the pre-treated liquid 1 of Example 6 treated with light basic magnesium carbonate, and the pre-treated liquid 2 of Comparative example 1 treated with a depth filter and an ultrafiltration membrane.
Figure 10 is a graph to show the infectivity titers of the adeno-associated virus purified by affinity chromatography from a purified AAV liquid, the pre-treated liquid 3 and the pre-treated liquid 4 of Example 11 treated by light basic magnesium carbonate, and the pre-treated liquid 5 of Comparative example 2 treated with a depth filter and an ultrafiltration membrane.

### MODE FOR CARRYING OUT THE INVENTION

Each of step of the present invention method is hereinafter described, and the present invention is not restricted to the following specific examples.

### 1. Preparation step of virus/virus-like particle-containing liquid

The liquid containing a virus or a virus-like particle to be subjected to purification is prepared in this step. This step may be arbitrarily implemented, and when the liquid containing a virus or a virus-like particle is already obtained, this step is not needed to be implemented.

A virus-like particle is mainly all or a part of a virus outer shell protein that constitutes a capsid. Since a virus-like particle does not contain a nucleic acid, a virus-like particle is not contagious. On the one hand, since a virus-like particle causes an immune reaction, a virus-like particle can be used as an active ingredient of vaccine. The "virus or virus-like particle" in this disclosure means not only "either of a virus or a virus-like particle" but also "both of a virus and a virus-like particle" in the case where both of a virus and a virus-like particle may be produced in the preparation step and a virus and a virus-like particle may be mixed.

The virus is not particularly restricted as long as the virus itself or a part of the virus should be purified. An example of a non-enveloped virus includes adeno-associated virus, adeno virus, enterovirus, parvovirus, papovavirus, human papilloma virus, rotavirus, coxsackievirus, sapovirus, norovirus, poliovirus, echovirus, hepatitis A virus, hepatitis E virus, rhinovirus and astrovirus. Adeno-associated virus has an AAV capsid serotype selected from the group consisting of AAV-1, AAV-2, AAV-3, AAV-4, AAV-5, AAV-6, AAV-7, AAV-8, AAV-9, AAV-10, AAV-11, AAV-12, AAV-13, AAV-14, AAV-15 and AAV-16. An example of an enveloped virus includes retrovirus, lentivirus, Sendai virus, herpes simplex virus, vaccinia virus, measles virus, baculovirus and influenza virus.

The liquid containing a virus may be produced by an ordinary method. For example, a hen egg is disinfected and then incubated for 10 to 12 days. A specific amount of a virus strain is inoculated into an allantoic cavity and cultivated for 2 to 3 days. Next, the egg is cooled for about half a day to bring viral proliferation to a stop. Then, a chorioallantoic fluid in which a virus is proliferated can be used as the virus-containing liquid.

The virus-containing liquid can be produced by a transformation method similarly to a virus-like particle-containing liquid. Specifically, a nucleic acid that encodes a virus or a virus-like particle is introduced into a host cell using a vector containing the nucleic acid to transform the host cell, and the transformed cell is cultivated. Then, the transformed cell is separated from a culture medium by centrifugation or filtration, and the separated cell is disrupted in a buffer solution containing a surfactant and the like. The obtained disrupted cell suspension may be treated with a nuclease to decompose the nucleic acid derived from the host cell; on the one hand, a usage amount of an expensive nuclease can be reduced, since an impurity can be efficiently removed by the present invention method. The obtained disrupted cell suspension is subjected to centrifugation or filtration, and the obtained supernatant can be used as the virus-containing liquid or the virus-like particle-containing liquid. The concentration of the virus or the virus-like particle in the virus-containing liquid or the virus-like particle-containing liquid is preferably adjusted to 10⁴ vg/mL or more and 10¹⁵ vg/mL or less using a buffer solution or the like.

A conventionally known cell can be used as a host cell to be transformed. For example, an example of such a conventionally known cell includes an animal cell such as HEK293 cell, CHO cell, COS cell, HeLa cell, C127 cell, 3T3 cell and BHK cell; an insect cell such as S2 cell and Sf cell; a bacterial cell such as Escherichia coli, Bacillus subtilis and bacteria of genus Bacillus; a fungus cell such as yeast and aspergillus; and a plant cell.

### 2. Contact step with water-insoluble inorganic compound

The virus or virus-like particle is purified in this step by contacting a liquid containing the virus or virus-like particle with a water-insoluble inorganic compound containing one or more elements selected from magnesium, calcium and aluminum to selectively adsorb an impurity other than the virus and virus-like particle on the water-insoluble inorganic compound. The term "purification" means that a ratio of an impurity to the virus or virus-like particle in the liquid before the liquid is contacted with the water-insoluble inorganic compound is reduced.

The term "water-insoluble" in this disclosure means that a degree to which an inorganic compound powder is dissolved within 30 minutes under a condition where the powder is added in water and the mixture is strongly shaken at 20 ± 5°C for 30 seconds every 5 minutes, and specifically means an amount of water required to dissolve 1 g of the inorganic compound is 400 mL or more.

The water-insoluble inorganic compound contains one or more elements selected from magnesium, calcium and aluminum, is exemplified by an insoluble carbonate salt, an insoluble sulfate salt and an oxide, and is preferably one or more selected from magnesium carbonate, magnesium hydroxide, magnesium oxide, calcium sulfate and aluminum oxide. For example, basic magnesium carbonate, which is a mixture of magnesium hydroxide and magnesium carbonate, is also preferably used. A phosphate salt is, however, not preferred, since such a phosphate salt has relatively high water-solubility and may possibly adsorb the target virus or virus-like particle.

The size of the water-insoluble inorganic compound may be appropriately adjusted, and for example, the average particle diameter may be adjusted to 0.1 µm or more and 1000 µm or less. When the average particle diameter is 1000 µm or less, the water-insoluble inorganic compound can adsorb an impurity more efficiently due to the sufficiently large specific surface area. When the average particle diameter is 0.1 µm or more, excessive energy for breakage is not needed. The average particle diameter is preferably 10 µm or more from the viewpoint of the handling property at the time of the filling in a column. The average particle diameter in this disclosure is measured by a laser diffraction particle size distribution measurement device. Volume, weight, number or the like are known as the base of an average particle diameter, and volume is preferred.

The shape and the structure of the water-soluble inorganic compound are not restricted, and for example, particle, flake, needle and tube can be used. The porous water-insoluble inorganic compound is advantageous to the removal of an impurity due to the large specific surface area thereof. For example, γ alumina, tubular basic magnesium carbonate, and spherical basic magnesium carbonate (see JP 2008-137827 A), which is an aggregate of a flower petal crystal, can be used. γ Alumina is cubical crystal alumina having a high specific surface area. Tubular basic magnesium carbonate means a micro tubular particle composed by aggregating foliaceous fine crystals of basic magnesium carbonate, and is exemplified by "MgTube^{(R)}" manufactured by Nittetsu Mining.

A usage amount of the water-insoluble inorganic compound may be adjusted depending on the concentration of the virus-containing liquid or the virus-like particle-containing liquid, and for example, 1 g or more and 20 g or less of the water-insoluble inorganic compound to 100 mL of the virus-containing liquid or the virus-like particle-containing liquid may be used. The ratio is preferably 15 g/100 mL or less. In addition, 1 mass% or more and 20 mass% or less of the water-insoluble inorganic compound to the virus-containing liquid or the virus-like particle-containing liquid is preferably used, and the ratio is preferably 15 mass% or less.

A method for contacting the virus-containing liquid or the virus-like particle-containing liquid with the water-insoluble inorganic compound may be appropriately selected. For example, the water-insoluble inorganic compound may be added to the virus-containing liquid or the virus-like particle-containing liquid, and the mixture may be shaken or stirred. The temperature at the time may be an atmospheric temperature, and may be specifically adjusted to 1°C or higher or 30°C or lower or 15°C or higher or 25°C or lower. The contact time may be adjusted to 5 seconds or more and 10 hours or less.

The water-insoluble inorganic compound may be separated from the virus-containing liquid or the virus-like particle-containing liquid by centrifugation or filtration after the contact. The virus or the virus-like particle is mainly dispersed in the liquid part and all of or a part of the other impurity is mainly adsorbed on the water-insoluble inorganic compound at the time. Though a part of the virus or the virus-like particle may be adsorbed on the water-insoluble inorganic compound and a part of the other impurity may be dissolved in the liquid part in some cases, at least a total amount of the impurity in the liquid part can be reduced and the virus or the virus-like particle can be concentrated in the liquid part.

An impurity that is adsorbed on the water-insoluble inorganic compound in this step is not particularly restricted as long as the impurity is not the virus and the virus-like particle, and is exemplified by a disrupted virus, a disrupted virus-like particle, a contaminant derived from a host cell and a contaminant derived from cell culture. An example of such a contaminant derived from a host cell includes a nucleic acid derived from a host cell, plasmid and a protein derived from a host cell. An example of such a contaminant derived from cell culture includes a culture medium component, serum albumin, the other serum protein and a plasmid DNA for transfection.

A column may be filled with the water-insoluble inorganic compound and the virus-containing liquid or the virus-like particle-containing liquid may be passed through the column to adsorb an impurity other than the virus or the virus-like particle on the water-insoluble inorganic compound. The adsorption of an impurity and the separation of the liquid part from the water-insoluble inorganic compound can be simultaneously carried out in such a case. An amount of the water-insoluble inorganic compound to fill a column and a flow rate of the virus-containing liquid or the virus-like particle-containing liquid are preferably adjusted within the range that an impurity is sufficiently adsorbed on the water-insoluble inorganic compound.

A filter having a layer containing the water-insoluble inorganic compound may be prepared and the virus-containing liquid or the virus-like particle-containing liquid may be filtrated to adsorb an impurity other than the virus or the virus-like particle on the filter. The water-insoluble inorganic compound may be merely deposited on a support material as a water-insoluble inorganic compound-containing layer or a water-insoluble inorganic compound-containing layer may be sandwiched between support materials from the upper and the lower sides. An example of a raw material of the support material includes one or more water-insoluble media selected from an activated carbon; a polysaccharide such as cellulose, cellulose acetate, nitrocellulose, agarose and chitosan; a synthetic polymer such as polyacrylonitrile, polyester, polyether sulfone, polypropylene and polytetrafluoroethylene; and an inorganic substance such as diatomite, pearlite, glass, silica, alumina, zirconia and barium titanate.

### 3. Step to contact with activated carbon

The liquid containing the virus or the virus-like particle is contacted with an activated carbon in this step. This step may be carried out before or after the above-described step to contact with the water-insoluble inorganic compound, or the steps may simultaneously be carried out by using the water-insoluble inorganic compound and an activated carbon in combination.

An activated carbon is obtained by burning a charcoal, a palm shell or the like to develop a pore to be porous, and is excellent in adsorption performance. A general specific surface area of an activated carbon is about 800 m²/g or more and about 2500 m²/g or less.

An example of an activated carbon includes a mineral activated carbon and a plant activated carbon. An example of such a mineral activated carbon includes a coal activated carbon and a petroleum activated carbon. An example of such a plant activated carbon includes a wood activated carbon and a palm shell activated carbon, and a wood activated carbon is preferred.

A raw material of an activated carbon is not particularly restricted as long as the raw material is a carbonaceous substance, and is exemplified by a woodiness such as sawdust, charcoal, ash, herbaceous peat, peat and wood chip; palm shell; a coal such as lignite, brown coal and anthracite; petroleum pitch; and an organic compound such as rayon, acrylonitrile and a phenolic resin.

A method for producing an activated carbon is not particularly restricted and is exemplified by a chemical liquid activation method and a gas activation method. In a chemical liquid activation method, zinc chloride, phosphoric acid or the like is added to a raw material at high temperature and a mixture is subjected to carbonization reaction at high temperature. In a gas activation method, a mixture of a carbonized raw material and a gas such as water vapor, carbon dioxide, air and combustion gas is reacted at high temperature. An example of the method preferably includes a zinc chloride activation method, an acid activation method using phosphoric acid, and a water vapor activation method.

A figure of an activated carbon is not particularly restricted, and an example of an activated carbon includes a granular activated carbon such as pulverized charcoal, granular charcoal, spherical charcoal and pellet charcoal; a fibrous activated carbon such as fiber and cloth; a specially formed activated carbon such as sheet, formed body and honeycomb; and a powder activated carbon.

Also, an activated carbon to which a charge of plus or minus is added and an activated carbon which is modified with a surface-modifying agent such as poly(hydroxyethyl methacrylate) (PHEMA), heparin, cellulose and polyurethane can be used in the purification method of the present invention. In addition, a carbon gel prepared by sol-gel method is included in the activated carbon usable in the purification method of the present invention. An example of a raw material used in the sol-gel method includes phenol, melamine, resorcinol and formaldehyde.

An average pore diameter of an activated carbon is not particularly restricted, and is generally 0.1 nm or more and 20 nm or less, preferably 0.5 nm or more and 5.0 nm or less, more preferably 2.0 nm or more and 5.0 nm or less, and even more preferably 3.0 nm or more and 5.0 nm or less. An average pore diameter of an activated carbon can be calculated from an adsorption isothermal curve of nitrogen gas using BJH method.

A means of a purification method using an activated carbon according to the present invention is not particularly restricted, and is exemplified by a batch method, a membrane treatment method and a column chromatography method. An appropriate figure of an activated carbon is selected depending on each of means. An activated carbon may be used in the forms of a particle prepared by enclosing an activated carbon in porous polymer or gel, a membrane prepared by adsorbing, immobilizing or forming an activated carbon using a fiber or a supporting agent such as polypropylene and cellulose, and a cartridge, as needed.

A membrane or a cartridge containing an activated carbon is specifically exemplified by CUNO^{(R)} activated carbon filter cartridge and Zeta Plus^{(R)} activated carbon filter cartridge (manufactured by Sumitomo 3M); Millistak+^{(R)} activated carbon filter (manufactured by Merck Millipore); Supra AKS1 filter, AKS1 filter, Stax^{™}, AKS1 (manufactured by Pall); Ad'All (manufactured by UNITIKA); K filter^{(R)}, activated carbon sheet (manufactured by TOYOBO); Hemax (manufactured by Kuraray); Hemosorba^{(R)} (manufactured by Asahi Kasei Medical); Hemocolumn (manufactured by TERUMO); and Hemocells (manufactured by TEIJIN), and is not restricted thereto. An example of a membrane or a cartridge containing a wood-based activated carbon among the above examples includes Zeta Plus^{(R)} activated carbon filter cartridge (manufactured by Sumitomo 3M); Supra AKS1 filter, AKS1 filter, Stax^{™} and AKS1 (manufactured by Pall).

A packing density, a particle size, a hardness, a weight loss on drying, an ignition residue, a specific surface area, a pore volume or the like of an activated carbon to be used can be appropriately selected.

A usage amount of an activated carbon may be adjusted depending on a concentration or the like of the virus-containing liquid or the virus-like particle-containing liquid, and for example, 0.5 g or more and 5 g or less of an activated carbon to 100 mL of the virus-containing liquid or the virus-like particle-containing liquid may be used.

As a method for contacting the virus-containing liquid or the virus-like particle-containing liquid with an activated carbon, similarly to the case of the water-insoluble inorganic compound, an activated carbon may be added to the virus-containing liquid or the virus-like particle-containing liquid and the mixture may be shaken or stirred, or a column may be filled with an activated carbon. When this step and the above-described step to contact with the water-insoluble inorganic compound are simultaneously carried out, the water-insoluble inorganic compound and an activated carbon may be mixed to be used.

### 4. Further purification step

The above-described step to contact with the water-insoluble inorganic compound or the combination of the step to contact with water-insoluble inorganic compound and the step to contact with an activated carbon may be repeated two or more times in the case where the virus or the virus-like particle is not sufficiently purified by one time implementation. The upper limit of the repeat number is not particularly restricted, and for example, may be 10 times or less and is preferably 5 times or less. In addition, when the virus or the virus-like particle is not sufficiently purified, a conventional purification method may be carried out after the step to contact with the water-insoluble inorganic compound or the combination of the step to contact with an activated carbon and the step to contact with the water-insoluble inorganic compound. A load on a conventional purification method can be remarkably reduced in such a case, since a concentration of an impurity can be reduced and the target virus or the virus-like particle can be concentrated by the step of the present invention. An example of a conventional purification method includes an ultracentrifugal method, membrane separation and chromatography, and membrane separation and chromatography are preferred in the view point of mass production. In addition, a nucleic acid that is contained in the liquid containing the virus or the virus-like particle and that is derived from a host may be treated by a nuclease to be decomposed after the step to contact with the water-insoluble inorganic compound. On the one hand, a usage amount of an expensive nuclease can be reduced by the present invention method, since an impurity can be effectively removed by the present invention.

Chromatography is exemplified by affinity chromatography and ion exchange chromatography, and is particularly preferably affinity chromatography. Since a pH and a salt concentration of an eluent are not needed to be preliminarily adjusted in affinity chromatography, purification by affinity chromatography is efficient.

In general, there are a small amount of virus and a large amount of an impurity in a cell culture fluid; therefore, when the virus or the virus-like particle is purified by chromatography, an impurity is preliminarily removed by ultrafiltration and the virus or the virus-like particle is preliminarily concentrated. On the one hand, a virus or a virus-like particle can be purified by chromatography without preliminary ultrafiltration after the liquid containing a virus or a virus-like particle is treated with the water-insoluble inorganic compound according to the present invention.

After a virus or a virus-like particle is purified, the virus or the virus-like particle may be concentrated by reducing an amount of a solvent or a solvent may be exchanged.

A virus or a virus-like particle having higher purity can be efficiently produced by the above-described purification method of the present invention.

The present application claims the benefit of the priority dates of Japanese patent application No. 2019-125243 filed on July 4, 2019. All of the contents of the Japanese patent application No. 2019-125243 filed on July 4, 2019, are incorporated by reference herein.

### EXAMPLES

The examples are hereinafter described to demonstrate the present invention more specifically, but the present invention is in no way restricted by the examples, and the examples can be appropriately modified to be carried out within a range which adapts to the contents of this specification. Such a modified example is also included in the range of the present invention.

### Example 1

### (1) Preparation of adeno-associated virus (AAV)-producing cell

A plasmid that produced AAV2 and that expressed VENUS (GenBank:ACQ43955.1), which is an altered fluorescent protein GFP, was prepared using AAV vector preparation kit ("AAVpro^{(R)} Helper Free System" manufactured by Takara Bio).

The cultured HEK293 cell was transfected with the prepared plasmid using transfection reagent ("Polyethylenimine MAX" manufactured by Polysciences, MW: 40,000) to produce AAV. The cell was released after the cultivation, and the cell culture fluid was recovered. The cell culture fluid was centrifuged to remove the supernatant and to obtain AAV-producing cell.

### (2) Evaluation of ability of magnesium salt to remove impurity protein

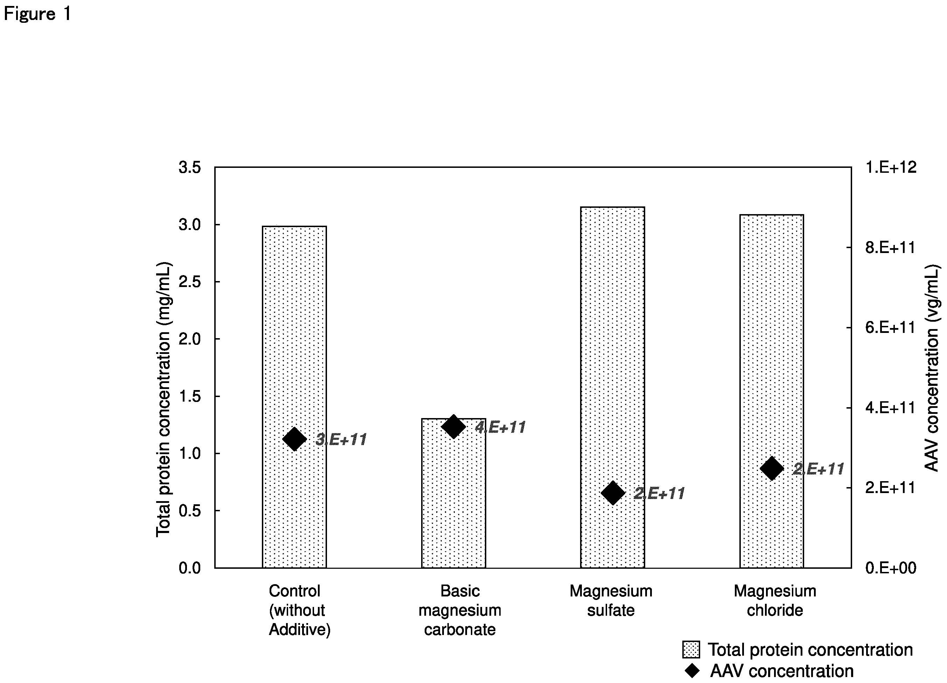

The AAV-producing cell obtained as the above (1) was suspended in Dulbecco's Phosphate Buffered Saline (manufactured by Sigma-Aldrich, hereinafter abbreviated as "PBS") containing 0.1% Triton^{(R)} X-100, and the suspension was stirred in ice for 20 minutes to disrupt the cell. To the obtained disrupted cell suspension, 1 M magnesium chloride aqueous solution was added in a concentration of 7.5 v/v% and 25 KU/mL endonuclease aqueous solution (manufactured by KANEKA) was added in a concentration of 0.1v/v%. The mixture was allowed to stand still at 37°C for 30 minutes to decompose the nucleic acid derived from the cell. After the reaction, 0.5 M EDTA solution was added to the reaction mixture in a concentration of 15 v/v%. Then, the mixture was centrifuged to recover the supernatant. The obtained supernatant was referred to as the pre-treated liquid. Into the pre-treated liquid, 100 v/v% of PBS to the pre-treated liquid, and 20 w/v% of magnesium sulfate heptahydrate, magnesium chloride or light basic magnesium carbonate (manufactured by Wako Pure Chemical Industries) to the pre-treated liquid were respectively added. The mixture was shaken at 25°C for 1 hour. The pre-treated liquid was diluted with PBS without adding an additive thereto, and the diluted liquid was similarly shaken as control. The mixture was centrifuged after shaking to obtain supernatant, and the amount of AAV and the mass of total proteins were measured. The AAV concentration was measured using AAV titer measurement kit ("AAVpro^{(R)} Titration Kit (for Real Time PCR) Ver. 2" manufactured by Takara Bio), and the total protein concentration was measured using BSA as a standard and protein colorimetric assay reagent ("Pierce 660 nm Protein Assay Reagent" manufactured by Thermo Fisher Scientific). The measurement result is shown in Table 1 and Figure 1.

**Table 1**

| Additive | AAV concentration | Total protein concentaration |
|---|---|---|
| | (vg/mL) | (mg/mL) |
| Control (without additive) | 3.2E+11 | 2.99 |
| Basic magnesium carbonate | 3.5E+11 | 1.30 |
| Magnesium sulfate | 1.9E+11 | 3.15 |
| Magnesium chloride | 2.5E+11 | 3.09 |

The total protein concentration was low but the AAV concentration was high in the liquid into which basic magnesium carbonate as a water-insoluble magnesium salt was added in accordance with the result shown in Table 1 and Figure 1. It was found by the result that an impurity protein can be remarkably removed and AAV can be recovered without loss by using a water-insoluble magnesium salt.

On the one hand, an impurity protein reducing effect could not be confirmed in the control case in which an additive was not added and the cases in which magnesium sulfate heptahydrate or magnesium chloride as a water-soluble magnesium salt was added. In addition, AAV concentration was low in the case where a water-soluble magnesium salt was added.

### Example 2: Evaluation of effect of additive amount of basic magnesium carbonate on impurity protein removal rate

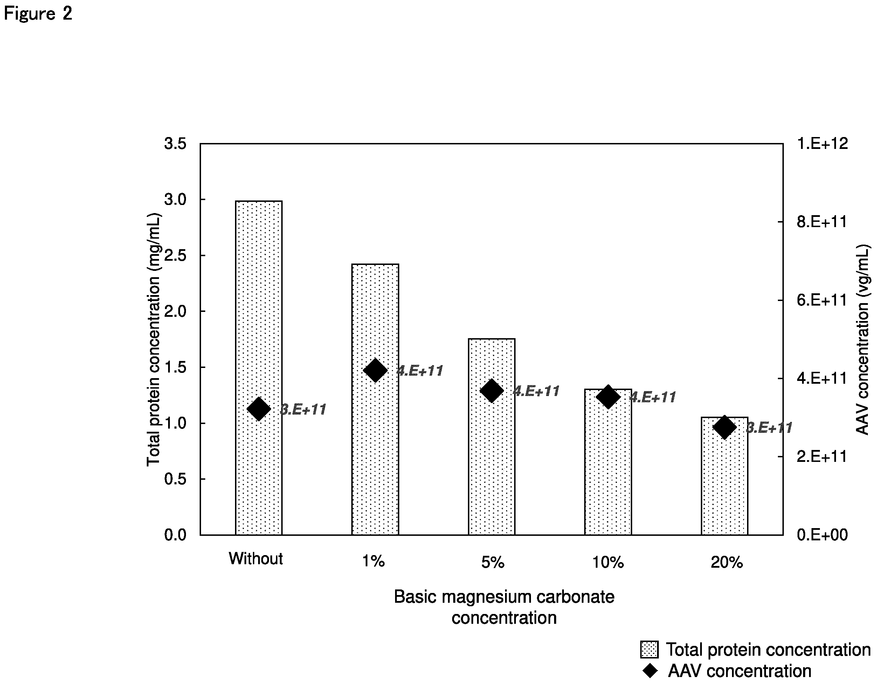

The pre-treated liquid was prepared similarly to Example 1, and PBS was added thereto in a ratio of 100 v/v%. Then, 1, 5, 10 or 20 w/v% of basic magnesium carbonate to the volume of each solution was added, and the AAV amount and the mass of total proteins were measured. In addition, the pre-treated liquid was diluted using PBS without adding an additive as control and similarly evaluated. The result is shown in Table 2 and Figure 2.

**Table 2**

| Additive amount of basic magnesium carbonate (w/v%) | AAV concentration | Total protein concentaration |
|---|---|---|
| | (vg/mL) | (mg/mL) |
| Without addition | 3.2E+11 | 2.99 |
| 1% | 4.2E+11 | 2.42 |
| 5% | 3.7E+11 | 1.75 |
| 10% | 3.5E+11 | 1.30 |
| 20% | 2.8E+11 | 1.05 |

When the additive amount of basic magnesium carbonate was larger in the range of 1 to 20 w/v% to the solution, the total protein concentration was low and the effect to reduce an impurity was high as the result shown in Table 2 and Figure 2. In addition, when an additive amount of basic magnesium carbonate was larger, AAV concentration tended to be lower; however, AAV with higher concentration than that without the additive could be obtained in the case of the concentration of 10 w/v% or less.

### Example 3: Evaluation of effect of salt concentration on impurity protein removal rate

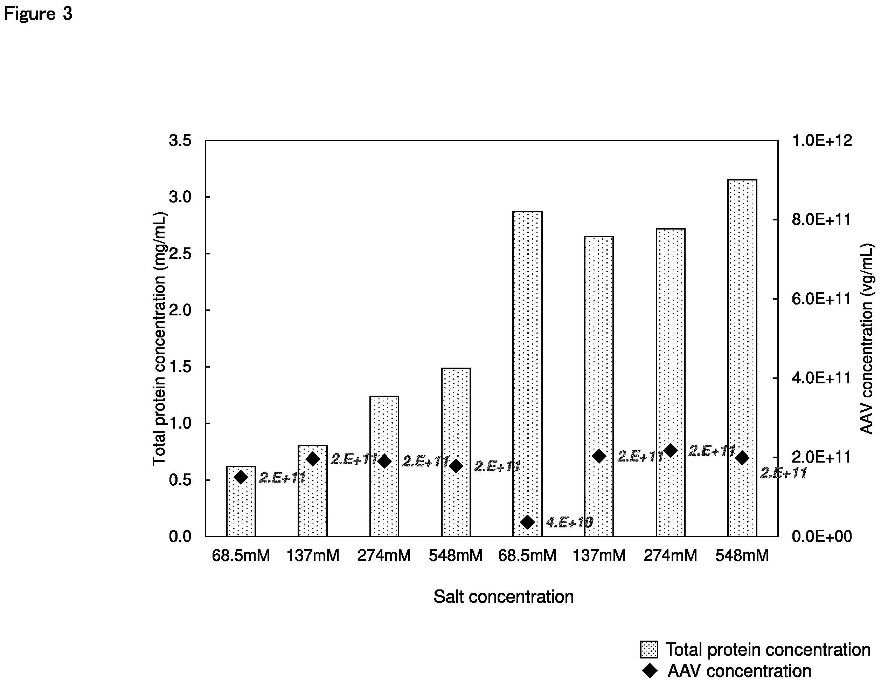

A phosphate buffer of pH 7.4 (0.2 g/L dipotassium hydrogen phosphate, 2.9 g/L disodium hydrogen phosphate-dodecahydrate) and 1 M sodium chloride-phosphate buffer of pH 7.4 (0.2 g/L dipotassium hydrogen phosphate, 2.9 g/L disodium hydrogen phosphate·dodecahydrate, 58.4 g/L sodium chloride) were prepared. The pre-treated liquid was prepared similarly to Example 1, and AAV solution having a final concentration of sodium chloride of 68.5 mM, 137 mM, 274 mM or 548 mM was prepared using the above-described 2 kinds of phosphate buffers in place of 100 v/v% of PBS. Basic magnesium carbonate was added to the solution in a concentration of 10 mass%, and the AAV amount and the mass of total proteins were measured. In addition, the pre-treated liquid was diluted using PBS without adding basic magnesium carbonate as control and similarly tested. The result is shown in Table 3 and Figure 3.

**Table 3**

| Additive | NaCl concentaration of buffer | AAV concentration | Total protein concentaration |
|---|---|---|---|
| | (mM) | (vg/mL) | (mg/mL) |
| Basic magnesium carbonate | 68.5 mM | 1.5E+11 | 0.62 |
| | 137 mM | 2.0E+11 | 0.80 |
| | 274 mM | 1.9E+11 | 1.24 |
| | 548 mM | 1.8E+11 | 1.49 |
| Control (without additive) | 68.5 mM | 3.7E+10 | 2.87 |
| | 137 mM | 2.0E+11 | 2.65 |
| | 274 mM | 2.2E+11 | 2.72 |
| | 548 mM | 2.0E+11 | 3.15 |

When the sodium chloride concentration before basic magnesium carbonate was added was lower in the sodium chloride concentration range of 68.5 to 548 mM, total protein concentration tended to be lower and the effect to reduce an impurity was higher as the result shown in Table 3 and Figure 3. In addition, AAV concentration did not seem to correlate with sodium chloride concentration.

### Example 4: Impurity removal effect by combination of water-insoluble inorganic compound and activated carbon

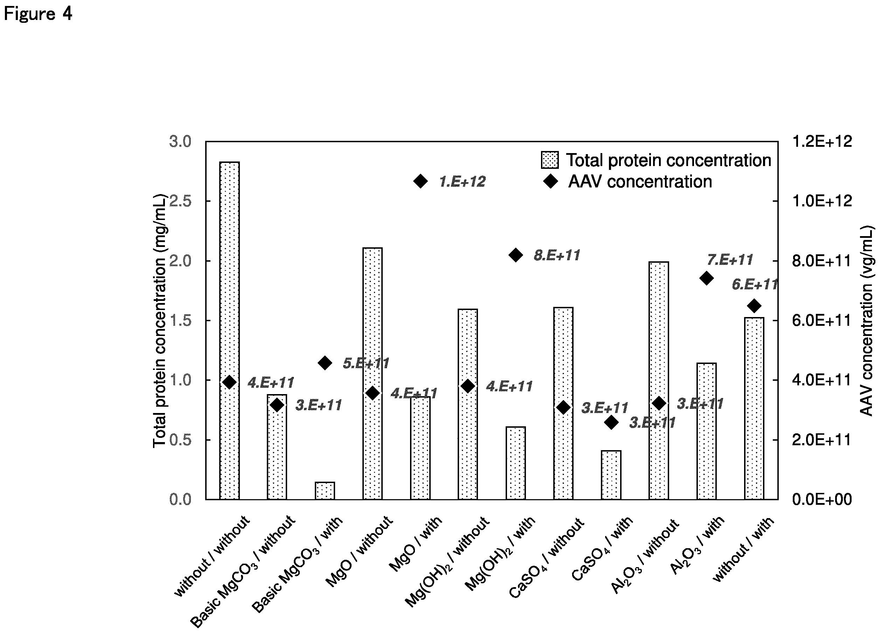

The pre-treated liquid was prepared similarly to Example 1, and 100 v/v% of PBS or PBS containing 2 mass% of activated carbon was added thereto. Then, 10 mass% of water-insoluble inorganic compound to the volume of each solution was added, and the AAV amount and the mass of total proteins were measured. Basic magnesium carbonate, magnesium oxide, magnesium hydroxide, calcium sulfate or aluminum oxide was used as a water-insoluble inorganic compound. In addition, the pre-treated liquid was diluted using PBS without adding a water-insoluble inorganic compound as control and similarly tested. The result is shown in Table 4 and Figure 4.

**Table 4**

| Additive | Activated carbon | AAV concentration | Total protein concentaration |
|---|---|---|---|
| | | (vg/mL) | (mg/mL) |
| None | without | 3.9E+11 | 2.83 |
| Basic magnesium carbonate | without | 3.2E+11 | 0.88 |
| Basic magnesium carbonate | with | 4.6E+11 | 0.14 |
| Magnesium oxide | without | 3.6E+11 | 2.11 |
| Magnesium oxide | with | 1.1E+12 | 0.86 |
| Magnesium hydroxide | without | 3.8E+11 | 1.59 |
| Magnesium hydroxide | with | 8.2E+11 | 0.61 |
| Calcium sulfate | without | 3.1E+11 | 1.61 |
| Calcium sulfate | with | 2.6E+11 | 0.41 |
| Aluminum oxide | without | 3.2E+11 | 1.99 |
| Aluminum oxide | with | 7.4E+11 | 1.14 |
| None | with | 6.5E+11 | 1.52 |

An impurity protein removal effect of not only basic magnesium carbonate but also the other water-insoluble inorganic compound of magnesium oxide, magnesium hydroxide, calcium sulfate and aluminum oxide was confirmed as the result shown in Table 4 and Figure 4. Any of the compounds are inorganic compounds that are used for a pharmaceutical product and medical use and that are suitable for producing a bio-pharmaceutical product. In addition, it was found that an impurity removal effect becomes higher in combination with an activated carbon.

### Example 5: Effect to reduce usage amount of endonuclease by water-insoluble inorganic compound and activated carbon

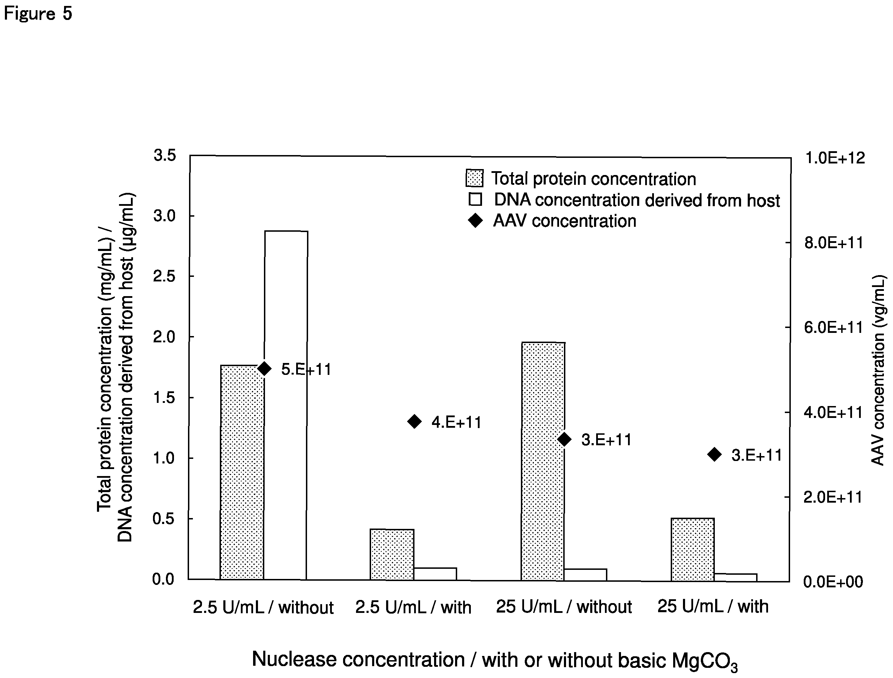

The AAV-producing cell obtained in Example 1(1) was suspended in PBS containing 0.1% Triton^{(R)} X-100. The suspension was stirred in ice for 20 minutes to disrupt the cell. To the obtained disrupted cell suspension, 1 M magnesium chloride solution in a concentration of 75 v/v% and 250 U/mL nuclease aqueous solution ("KANEKA Endonuclease" manufactured by KANEKA) in a concentration of 0.1 v/v% or 0.01 v/v% were added. The mixture was allowed to stand at 37°C for 30 minutes to decompose the nucleic acid derived from the cell. After the reaction, 0.5 M EDTA was added to the reaction mixture in the concentration of 15 v/v% to obtain a pre-treated liquid. Two kinds of liquid were prepared without adding a water-insoluble inorganic compound to the pre-treated liquid or by adding basic magnesium carbonate only in a concentration of 10 mass%, and were shaken at 25°C for 1 hour. The treated liquid was centrifuged after stirring to obtain a supernatant. The AAV concentration and the total protein concentration in the supernatant were measured similarly to Example 1. The remaining DNA derived from HEK293 cell in the supernatant was measured in reference to the method described in J. Phrama. Biomed. Anal. (2014), 100, 145-149. Specifically, primer 1: GAGGCGGGCGGATCA (SEQ ID NO. 1), primer 2: CCCGGCTAATTTTTGTATTTTTAGTAG (SEQ ID NO. 2) and real-time PCR reagent set ("Power SYBR^{™} Green PCR Master Mix" manufactured by Life Technologies) were used for the analysis using QuantStudio3 real-time PCR system (manufactured by Life Technologies). A standard curve was prepared using Human Genomic DNA (manufactured by GenScript) as DNA standard product derived from human to determine an amount of DNA. The result is shown in Table 5 and Figure 5.

**Table 5**

| Enzyme concentration | Water-insoluble inorganic compound | AAV concentration | Total protein concentaration | Concentration of DNA derived from host |
|---|---|---|---|---|
| | | (vg/mL) | (mg/mL) | (µg/mL) |
| 2.5U/mL | without | 5.0E+11 | 1.77 | 2.88 |
| 2.5U/mL | with | 3.7E+11 | 0.42 | 0.10 |
| 25U/mL | without | 3.3E+11 | 1.97 | 0.10 |
| 25U/mL | with | 3.0E+11 | 0.52 | 0.06 |

When 25 U/mL of nuclease was used and when 2.5 U/mL of nuclease was used and basic magnesium carbonate was added, remaining amounts of DNA derived from the host were nearly equal as the result shown in Table 5 and Figure 5. It was found by the result that even when a usage amount of expensive nuclease is reduced to 1/10 amount, DNA derived from a host can be efficiently removed by using a water-insoluble inorganic compound.

### Example 6: Rough purification of virus

### (1) Preparation of adeno-associated virus (AAV)-producing cell

A plasmid that produced AAV and that expressed VENUS (GenBank: ACQ43955.1), which is a modified fluorescent protein GFP, was prepared using AAV vector production kit ("AAVpro^{(R)} Helper Free System" manufactured by Takara Bio). Any one of AAV1, AAV2, AAV5 and AAV6 in the kit was used as the serotype of AAV.

The cultivated HEK293 was transfected with the prepared plasmid using transfection reagent ("Polyethylenimine MAX" manufactured by Polysciences, MW: 40,000) to produce AAV. The cell was released after the cultivation to obtain the cell culture fluid.

### (2) Preparation of nucleic acid decomposition treatment liquid

A surfactant ("Triton^{(R)} X-100") was added to the AAV culture fluid obtained in the above (1) in a concentration of 0.1 v/v%, and the mixture was stirred in ice for 20 minutes to dissolve the cell. To the obtained cell lysate, 1 M magnesium chloride aqueous solution and endonuclease (250 KU/mL, manufactured by KANEKA) was added in concentrations of 7.5 v/v% and 0.1 v/v% respectively. The mixture was allowed to stand at 37°C for 30 minutes to decompose the nucleic acid derived from the cell. The resulting liquid was referred to as the nucleic acid decomposition treatment liquid. The AAV amount and the mass of total proteins in the liquid were measured. The result is shown in Table 6. The AAV concentration was measured using AAV titer measurement kit ("AAVpro^{(R)} Titration Kit (for Real Time PCR) Ver. 2" manufactured by Takara Bio), and the mass of the total proteins was measured using bovine serum albumin (BSA) as a standard and protein colorimetric assay reagent ("Pierce 660 nm Protein Assay Reagent" manufactured by Thermo Fisher Scientific).

### (3) Removal of impurity by water-insoluble magnesium compound

Light basic magnesium carbonate (manufactured by Wako Pure Chemical Industries, 59 g) and a stirrer bar were added into a cylindrical polyethylene vessel of outside diameter 12.7 cm × overall height 23.5 cm. The nucleic acid decomposition treatment liquid obtained in the above (2) (590 g) was further added, and the mixture was stirred at room temperature for 1 hour. The ratio of light basic magnesium carbonate to the nucleic acid decomposition treatment liquid was 10 w/w%.

Then, the above mixture was filrated using a polyether sulfone filter ("Nalgene Rapid-Flow Sterile Disposable Filter Units" manufactured by Thermo Scientific, pore diameter: 0.2 µm). The obtained filtrate was referred to as the first filtrate. PBS (59 mL) was added to the remaining residue and filtrated. The obtained filtrate was referred to as the second filtrate. The first filtrate and the second filtrate were mixed. The mixture was referred to as the pre-treated liquid 1, and the AAV amount and the mass of total proteins were measured similarly to the above. The AAV amount and the mass of total proteins in the nucleic acid decomposition treatment liquid were similarly measured. The result is shown in Table 6.

**Table 6**

| | Liquid weight (g) | Protein concentration (mg/mL) | AAV concentration (vg/mL) | AAV amount (vg) |
|---|---|---|---|---|
| Nucleic acid decomposition treatment liquid | 590 | 0.37 | 1.73E+10 | 1.02E+13 |
| Pre-treated liquid 1 (Example 6) | 605 | 0.02 | 1.98E+10 | 1.20E+13 |

A decrease of AAV amount after the treatment was not observed as the result shown in Table 6. On the one hand, a mass of total proteins was remarkably reduced. Thus, it was confirmed that an impurity protein derived from a cell can be easily removed by the present invention method.

In addition, it was confirmed that not only a cell lysate but also basic magnesium carbonate particle on which an impurity protein may be adsorbed can be separated from the target AAV using a 0.2 µm filter.

### (4) Particle size distribution measurement of water-insoluble inorganic compound

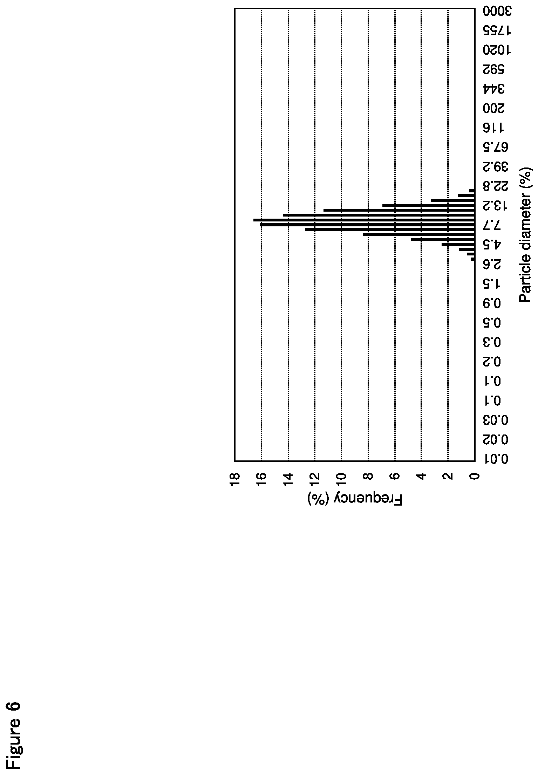

Light basic magnesium carbonate used in Example 6(3) was suspended in water, and particle size distribution was evaluated with a wet method using a particle size distribution measurement apparatus ("Partica LA-960" manufactured by HORIBA). The result is shown in Figure 6.

As a result, the median diameter was 7.9 µm and 10% diameter in the volume-based cumulative particle size distribution was 5.2 µm. In other words, when a filter having a pore diameter of 5 µm or less is used, 90% or more of the above basic magnesium carbonate may be separated. Since AAV passed through a filter having a pore diameter of 0.2 µm in Example 6(3), basic magnesium carbonate particle can be separated from AAV by using a filter of 0.2 to 5 µm.

### Comparative example 1: Rough purification of virus using depth filter and ultrafiltration membrane

A depth filter ("Supracap 50 capsule with V100P" manufactured by PALL, effective filtering area: 22 cm², pore diameter: 1 to 3 µm) was rinsed using 20 mM Tris + 120 mM NaCl aqueous solution (pH 8.0). Then, the nucleic acid decomposition treatment liquid obtained in Example 1(2) (592 g) was filtrated using the depth filter, and the obtained filtrate was referred to as the first filtrate. Further, 20 mM Tris + 120 mM NaCl aqueous solution (pH 8.0, 50 mL) was allowed to pass through the depth filter, and the obtained filtrate was referred to as the second filtrate. The first filtrate and the second filtrate were mixed to be the purified liquid.

The purified liquid (647 mL) was concentrated to about 50 mL using a pomp system ("AKTA flux S" manufactured by GE Healthcare) and an ultrafiltration membrane ("Suspended-Screen Ultrafiltration Cassettes with Omegatm Membrane: Centramate" manufactured by PALL, membrane area: 0.02 m², nominal molecular weight cut off: 300 K). The buffer was exchanged by continuously adding about 8 times the amount of 20 mM Tris + 120 mM NaCl + 0.005% Tween20 + 1 mM MgCl₂ aqueous solution (pH 8.1) as a dialysate to the concentrated liquid while the amount of the concentrated liquid was maintained. The dialyzed liquid was obtained with washing the inside of the system after the dialysis.

The dialyzed liquid was filtrated using a polyether sulfone filter ("Nalgene Rapid-Flow Sterile Disposable Filter Units" manufactured by Thermo Scientific, pore diameter: 0.2 µm), and the filtrate was referred to as the pre-treated liquid 2. The AAV amount and the mass of total proteins in the pre-treated liquid 2 were measured similarly to Example 6(1). The result is shown in Table 7.

**Table 7**

| | Liquid weight (g) | Protein concntration (mg/mL) | AAV concentration (vg/mL) | AAV amount (vg) |
|---|---|---|---|---|
| Nucleic acid decomposition treatment liquid | 592 | 0.37 | 1.73E+10 | 1.02E+13 |
| Purified liquid | 646 | 0.29 | 1.77E+10 | 1.15E+13 |
| Concentrated liquid | 577 | 0.22 | 8.57E+09 | 4.94E+12 |
| Dialyzed liquid | 422 | 0.03 | 8.38E+09 | 3.54E+12 |
| Pre-treated liquid 2 (Comparative example 1) | 102 | <0.01 | 2.49E+10 | 2.55E+12 |

The AAV amount was not changed, the concentration of the total proteins was not remarkably reduced and the most part of an impurity protein remained after the filtration using a depth filter as the result shown in Table 7.

An impurity protein could be removed as the total protein concentration was remarkably reduced but the AAV amount was also decreased after the treatment using an ultrafiltration membrane. In addition, AAV was also observed in the filtrate. AAV might pass through the membrane, since the pore diameter of the ultrafiltration membrane was too large.

It was found from the above result that crude purification using a depth filter and an ultrafiltration membrane requires multistep procedures and cumbersome, and a total protein mass can be reduced but there is a risk for the development of a decrease of AAV recovery rate by crude purification using a depth filter and an ultrafiltration membrane.

### Example 7: Purification of virus by affinity chromatography

The nucleic acid decomposition treatment liquid obtained in Example 6(2) was centrifuged, and the supernatant was filtrated using a polyether sulfone filter ("Nalgene Rapid-Flow Sterile Disposable Filter Units" manufactured by Thermo Scientific, pore diameter: 0.2 µm). The filtrate was referred to as the purified liquid without pre-treatment. The liquid was taken out from the purified liquid without pre-treatment in an amount in which 1 × 10¹² vg of AAV was contained, and 9 times volume of an equilibration buffer was mixed. The mixture was filtrated using a filter.

The obtained filtrate was subjected to affinity chromatography in the following condition to purify AAV. The amount of AAV in the solution was measured by quantitative PCR, and a recovery rate to the loaded AAV amount was calculated.

### Chromatography condition

Column: Tricron 5/50 (manufactured by GE Healthcare)
Carrier: POROS Capture Select AAVX (manufactured by Thermo Scientific), 1 mL
Flow rate: 0.5 mL/min
Equilibration buffer: 20 mM tris(hydroxymethyl)aminomethane - hydrochloride buffer, 0.5 M sodium chloride (pH 8.0)
Elution buffer: 0.1 M citrate buffer (pH 2.1)

Liquids were taken out from the pre-treated liquid 1 of Example 1 and the pre-treated liquid 2 of Comparative example 1 in an amount in which 1 × 10¹² vg of AAV was contained, and 9 times volume of an equilibration buffer was mixed. The mixture was filtrated using a filter. The filtrates were also subjected to affinity chromatography in the same condition to measure the amount of AAV and calculate recovery rate of AAV. The result is shown in Table 8.

**Table 8**

| | AAV amount before treatment (vg) | AAV amount after treatment (vg) | Recovery rate |
|---|---|---|---|
| Purified liquid without pre-treatment | 1.1E+12 | 6.0E+11 | 57% |
| Pre-treated liquid 1 (Example 6) | 8.0E+11 | 7.2E+11 | 90% |
| Pre-treated liquid 2 (Comparative example 1) | 8.6E+11 | 6.1E+11 | 71% |

It was found from the result of Table 8 that a recovery rate by affinity chromatography in the latter stage becomes higher in the case of the purified liquid obtained by the pre-treatment of the present invention example than those of the pre-treated liquid obtained by a depth filter and ultrafiltration (Comparative example 1) and the purified liquid without pre-treatment (Comparative example 2).

### Example 8: Evaluation of treatment condition using water-insoluble magnesium compound

The AAV culture fluid was treated by using water-insoluble magnesium compounds in the condition shown in Table 4, specifically additive amount, treatment time and nuclease concentration, to evaluate AAV recovery rate, protein removal rate and DNA removal rate.

A surfactant ("Triton^{(R)} X-100") was added to the AAV culture fluid obtained in Example 6(1) in a concentration of 0.1 v/v%, and the mixture was stirred in ice for 20 minutes to dissolve the cells. To the obtained cell dissolution liquid, 1 M magnesium chloride aqueous solution in a concentration of 7.5 v/v% and endonuclease (manufactured by KANEKA) in a final concentration of 50 U/mL, 5 U/mL or 0.5 U/mL were added. The mixture was allowed to stand at 37°C for 30 minutes to decompose the nucleic acid derived from the cell. The mixture was referred to as the nucleic acid decomposition treatment liquid. Light basic magnesium carbonate (manufactured by Wako Pure Chemical Industries) was added to 20 mL of each nucleic acid decomposition treatment liquid in a concentration of 10 w/v%, 5 w/v% or 1 w/v%, and the mixture was shaken and stirred at room temperature for 1 minute, 10 minutes or 60 minutes. The treated liquid was filtrated using a polyether sulfone filter ("Nalgene Rapid-Flow Sterile Disposable Filter Units" manufactured by Thermo Scientific, pore diameter: 0.2 µm). The obtained filtrate is hereinafter referred to the first filtrate. PBS (2 mL) was added to the filtration residue, and the mixture was filtrated. The obtained filtrate is hereinafter referred to the second filtrate. The first filtrate and the second filtrate were mixed. The mixture is hereinafter referred to as the pre-treated liquid 3. The AAV amount and the mass of total proteins in the pre-treated liquid 3 were measured in the same condition of Example 6(2). In addition, the remaining amount of the DNA derived from the host was measured by the same method of Example 5.

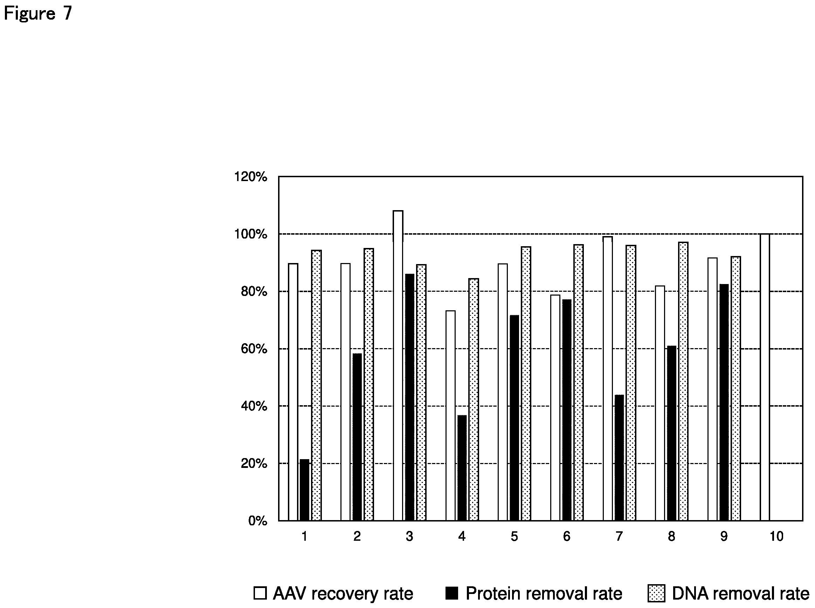

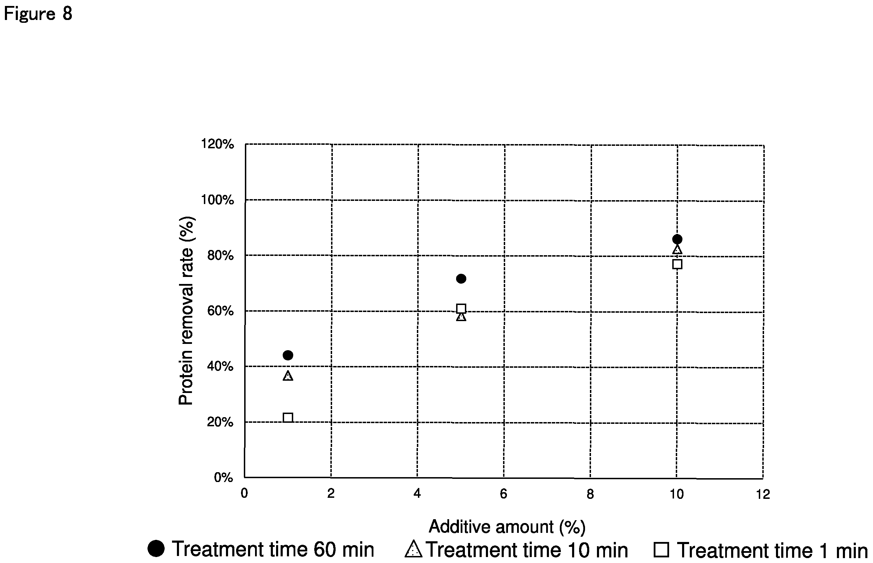

Furthermore, the AAV amount, the protein mass and the DNA amount in the nucleic acid decomposition treatment liquid treated with 50 U/mL of endonuclease without being treated with basic magnesium carbonate were measured in the same condition as control. In addition, the AAV recovery rate of each treated liquid in the case where the AAV amount of the control was taken as a base of 100% was calculated. Furthermore, the protein removal rate and the DNA removal rate were calculated on the assumption that the each removal rate about the protein mass and the DNA amount of the control was taken as 0%. The result is shown in Table 9, Figure 7 and Figure 8.

**Table 9**

| Test No. | Nuclease concentration (U/mL) | Treatment with water-insoluble inorganic compound | | AAV recovery rate | Protein removal rate | DNA removal rate |
|---|---|---|---|---|---|---|
| | | Additive amount | Treatment time | | | |
| 1 | 0.5 | 1% | 1min | 90% | 22% | 94% |
| 2 | 0.5 | 5% | 10min | 90% | 58% | 95% |
| 3 | 0.5 | 10% | 60min | 108% | 86% | 89% |
| 4 | 5 | 1% | 10min | 73% | 37% | 84% |
| 5 | 5 | 5% | 60min | 90% | 72% | 96% |
| 6 | 5 | 10% | 1min | 79% | 77% | 96% |
| 7 | 50 | 1% | 60min | 99% | 44% | 96% |
| 8 | 50 | 5% | 1min | 82% | 61% | 97% |
| 9 | 50 | 10% | 10min | 92% | 83% | 92% |
| Control | 50 | - | - | 100% | 0% | 0% |

It was found that 84% or more of removal rate was obtained in any conditions with respect to DNA removal rate, and DNA can be efficiently removed in each condition of additive amount, treatment time and nuclease concentration even at lowest standard. The protein removal rate tended to become higher in the case where the additive amount of basic magnesium carbonate was larger with respect to a protein concentration as shown in Figure 8. The larger additive amount is, therefore, preferred, in order to efficiently remove a protein.

### Example 9: Measurement of AAV particle diameter

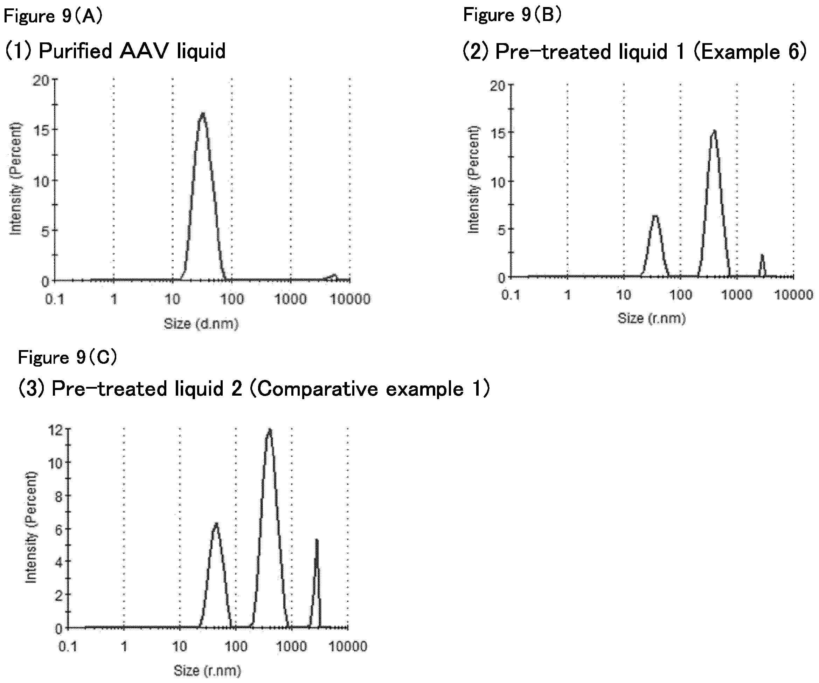

The particle size distributions of the pre-treated liquid 1 treated with light basic magnesium carbonate in Example 6, the pre-treated liquid 2 treated with a depth filter and an ultrafiltration membrane in Comparative example 1, and the purified AAV liquid purified with cation exchange chromatography and anion exchange chromatography were measured using a particle diameter / molecular size measurement device ("Zetasizer NanoZS" manufactured by Malvern). The result is shown in Table 10 and Figure 9.

**Table 10**

| | Particle diameter |
|---|---|
| Purified AAV | 32.67±10.96 nm |
| Pre-treated liquid 1 (Example 6) | 34.03±7.826 nm |
| Pre-treated liquid 2 (Comparative example 1) | 45.64±11.30 nm |

The peak estimated to be that of AAV was confirmed around 30 nm in the measurement result of particle size distribution for purified AAV. In addition, the peak of similar size was confirmed for the pre-treated liquid 1. It was assumed from the result that AAV can be purified as a particle having a similar size by removing an impurity with basic magnesium carbonate. On the one hand, the peak estimated to be that of AAV was confirmed around 40 nm in the measurement result of the pre-treated liquid 2. The reason why the particle diameter was larger than that of the purified AAV may be that the protein and the DNA derived from the host were adsorbed on the AAV particle and the AAV particle was observed as a large particle.

In addition, the reason why the purification yield of Example 7 by affinity chromatography from the pre-treated liquid 1 was higher than that from the pre-treated liquid 2 may be that the pore of the beads carrier was difficult to be filled with the AAV and the impurity due to the smaller size of AAV particle on which the impurity was not adsorbed.

### Example 10: Evaluation of infectivity titer of purified virus

The infectivity titers of AAV contained in the pre-treated liquid 1 treated with light basic magnesium carbonate in Example 6(3) and the pre-treated liquid 2 treated with a depth filter and an ultrafiltration membrane in Comparative example 1 were evaluated.

Specifically, AAV was purified by affinity chromatography from the pre-treated liquid 1 or the pre-treated liquid 2. HEK293 cell was cultivated and seeded in a rate of 4 × 10⁴ cells/well on 96 well plate coated using high purity collagen acidic solution ("AteloCell" manufactured by KOKEN). The cell was cultivated at 37°C overnight and attached on the coated plate. The AAV purified from the pre-treated liquid 1 or the pre-treated liquid 2 was diluted with Dulbecco's modified Eagle medium (DMEM, manufactured by Thermo Fisher Scientific). The medium was removed and replaced with each diluted AAV liquid to be cultivated at 37°C. The MOI (Multiplicity Of Infection) at the time was 10000. Each of the experiment was respectively carried out in 3 cases. The fluorescence intensity of fluorescent protein Venus produced by HEK293 cell infected by AAV was measured using Cytation1 cell imaging multimode reader (manufactured by BioTek) in the conditions of excitation wavelength of 485 nm and fluorescence wavelength of 528 nm after cultivation.

As a result, the fluorescence intensity in the case of the infection by AAV purified from the pre-treated 1 was 98392 ± 6247. When AAV was purified from the pre-treated 2, the fluorescence intensity was 72580 ± 23300. Thus, it was confirmed that there was not a significant difference in the fluorescence intensities and the AAV purified by the present invention method has a similar infectivity titer to that of the AAV purified by a conventional method.

### Example 11: Removal of impurity from culture of AAV having various serotypes

The cell dissolution liquids of AAV1, AAV2, AAV5 and AAV6 were obtained by the method described in Example 6. To 5 mL of each cell dissolution liquid, 50 mg of basic magnesium carbonate was added. The mixture was shaken at room temperature for 1 hour. Each cell dissolution liquid without an additive was also shaken in the same condition as control. The supernatant was obtained by centrifugation, and the AAV concentration, the protein concentration and the concentration of DNA derived from the host in the supernatant were measured. The result was shown in Table 11.

**Table 11**

| Serotype | Additive | AAV amount (vg) | Protein concentration (mg/mL) | DNA concentration (ng/mL) | AAV recovery rate (%) | Protein removal rate (%) | DNA removal rate (%) |
|---|---|---|---|---|---|---|---|
| AAV1 | - | 2.4E+11 | 0.21 | 7201 | 100 | 0 | 0 |
| | 1% | 1.7E+11 | 0.08 | 40 | 72 | 64 | 99 |
| AAV2 | - | 2.0E+11 | 0.18 | 4916 | 100 | 0 | 0 |
| | 1% | 1.9E+11 | 0.04 | 21 | 94 | 79 | 100 |
| AAV5 | - | 2.3E+11 | 0.16 | 3513 | 100 | 0 | 0 |
| | 1% | 1.8E+11 | 0.04 | 29 | 78 | 74 | 99 |
| AAV6 | - | 1.5E+11 | 0.21 | 6436 | 100 | 0 | 0 |
| | 1% | 1.6E+11 | 0.08 | 56 | 106 | 62 | 99 |

It was found from the result shown in Table 11 that a protein and DNA derived from a host can be efficiently removed by using a water-insoluble inorganic compound in any cases of serotypes. The present invention method can be considered to be broadly applicable regardless of a serotype of AAV on the basis of the above result.

### Example 12

A disrupted cell suspension was prepared in the same condition as Example 6, and endonuclease (250 kU/mL, manufactured by KANEKA) was added thereto in a final concentration of 5 U/mL. The mixture was allowed to stand at 37°C for 30 minutes to decompose the nucleic acid derived from the cell. The obtained nucleic acid decomposition treatment liquid (260 g) and light basic magnesium carbonate (manufactured by Wako Pure Chemical Industries, 2.6 g) were mixed, and the mixture was stirred at room temperature for 10 minutes. The ratio of light basic magnesium carbonate to the nucleic acid decomposition treatment liquid was 1 w/w%.

Then, the above mixture was filtrated using a polyether sulfone filter ("Nalgene Rapid-Flow Sterile Disposable Filter Units" manufactured by Thermo Scientific, pore diameter: 0.2 µm), and the obtained filtrate was referred to as the first filtrate. PBS (26 mL) was added to the filtration residue, and the mixture was filtrated. The obtained filtrate was referred to as the second filtrate. The first filtrate and the second filtrate were mixed, and the mixture was referred to as the pre-treated liquid 3. The AAV amount, the total protein mass and the remaining DNA concentration in the pre-treated liquid 3 were measured.

In addition, light basic magnesium carbonate (manufactured by Wako Pure Chemical Industries, 26 g) and the nucleic acid decomposition treatment liquid (264 g) were mixed, and the mixture was stirred at room temperature for 10 minutes. The pre-treated liquid 4 was similarly obtained, and the AAV amount, the total protein mass and the remaining DNA concentration were measured. The ratio of light basic magnesium carbonate to the nucleic acid decomposition treatment liquid was 10 w/w%. The result is shown in Table 12 and Table 13.

**Table 12**

| | Liquid weight | Protein concentration | DNA concentration | AAV concentration | AAV amount |
|---|---|---|---|---|---|
| Before treatment | 260g | 0.502mg/mL | 15.3ng/mL | 7.06E+10vg/mL | 1.84E+13vg |
| Pre-treated liquid 3 (Example 11) | 256g | 0.312mg/mL | 0.01ng/mL | 7.46E+10vg/mL | 1.91E+13vg |

**Table 13**

| | 'Liquid' weight | Protein concentration | DNA concentration | AAV concentration | AAV amount |
|---|---|---|---|---|---|
| Before treatment | 264g | 0.502mg/mL | 15.3ng/mL | 7.06E+10vg/mL | 1.86E+13vg |
| Pre-treated liquid 4 (Example 11) | 280g | 0.359mg/mL | 0.01ng/mL | 5.83E+10vg/mL | 1.63E+13vg |

### Comparative example 2

A disrupted cell suspension was prepared in the same condition as Example 6, and endonuclease (250 kU/mL, manufactured by KANEKA) was added thereto in a final concentration of 50 U/mL. The mixture was allowed to stand at 37°C for 30 minutes to decompose the nucleic acid derived from the cell. The nucleic acid decomposition liquid (532 g) was filtrated in the same condition as Comparative example 1 to obtain a purified liquid. The liquid was concentrated and the buffer was exchanged in the same condition as Comparative example 2 using a pump system ("AKTA flux S" manufactured by GE Healthcare) and an ultrafiltration membrane ("Suspended-Screen Ultrafiltration Cassettes with Omegatm Membrane: Centramate" manufactured by PALL, membrane area: 0.02 m², nominal molecular weight cut off: 100 K). The liquid was referred to as the pre-treated liquid 5, and the AAV amount, the total protein mass and the remaining DNA concentration were measured. The result is shown in Table 14.

**Table 14**

| | Liquid weight | Protein concentration | DNA concentration | AAV concentration | AAV amount |
|---|---|---|---|---|---|
| Before treatment | 532g | 0.502mg/mL | 15.3ng/mL | 7.06E+10vg/mL | 3.75E+13vg |
| Pre-treated liquid 5 (Comparative example 2) | 69g | 0.275mg/mL | 0.01ng/mL | 3.87E+11vg/mL | 2.67E+13vg |

It was found by the result shown in Tables 12 to 14 that even when an endonuclease amount is adjusted to 1/10 amount, a remaining DNA amount can be reduced to a similar degree to that of the pre-treated liquid 5 by the impurity removal method using light basic magnesium carbonate (pre-treated liquid 3 and pre-treated liquid 4).

### Example 13

AAV was purified from the pre-treated liquids obtained in Example 12 and Comparative example 2 using affinity chromatography by the same method as Example 7. The elution buffer was 0.1 M citrate + 0.5 M NaCl (pH 2.1).

The infectivity titer of the purified AAV was measured. HEK293 cell was cultivated and seeded in a rate of 4 × 10⁴ cells/well on 96 well plate coated using collagen for cell culture ("AteloCell" manufactured by KOKEN). The cell was cultivated at 37°C overnight and attached on the coated plate. The purified AAV was diluted with DMEM (manufactured by Thermo Fisher Scientific). The medium was removed and replaced with diluted AAV to be cultivated at 37°C. The MOI (Multiplicity Of Infection) at the time was 10000. Each of the experiment was respectively carried out in 3 cases. The fluorescence intensity of fluorescent protein Venus produced by HEK293 cell infected by AAV was measured using Cytation1 cell imaging multimode reader (manufactured by BioTek) in the conditions of excitation wavelength of 485 nm and fluorescence wavelength of 528 nm after cultivation.

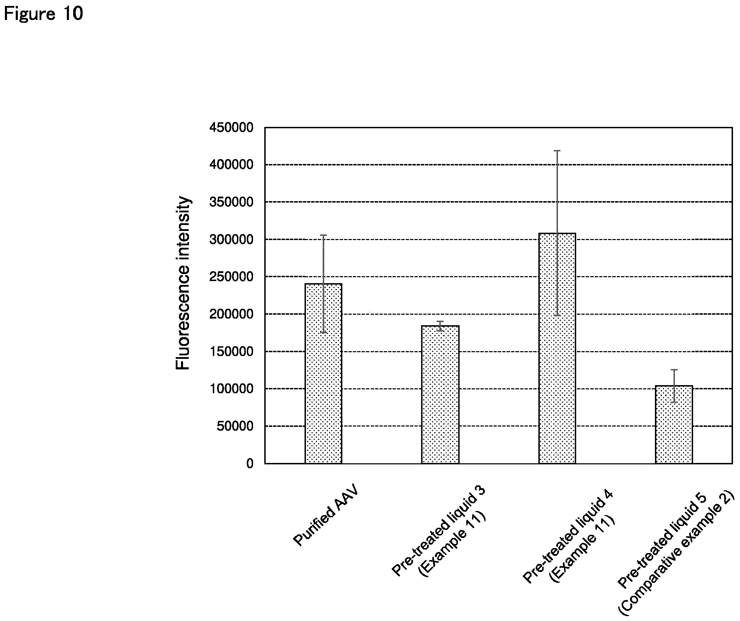

In addition, the infectivity titer of AAV purified by ultracentrifugation was evaluated as control. The result is shown in Table 15 and Figure 10.

**Table 15**

| Sample before chromatography | | Fluorescence intensity | |
|---|---|---|---|
| | | Average value | Standard error |
| Example 11 | Pre-treated liquid 3 | 240730 | 65381 |
| Example 11 | Pre-treated liquid 4 | 183836 | 6463 |
| Comparative example 2 | Pre-treated liquid 5 | 308309 | 110199 |
| Control | AAV purified by ultracentrifugation | 103941 | 21767 |

There was not a significant difference of fluorescence intensities and infectivity titers were similar between the cases where a cell was infected with AAV purified by subjecting the pre-treated liquid obtained in Example 12 to affinity chromatography and a cell was infected with AAV purified by an ultracentrifugation method.

On the one hand, the infectivity titer of the AAV purified by subjecting the pre-treated liquid obtained in Comparative example 2 to affinity chromatography might be low, since the fluorescence intensity thereof was lower than that of the AAV purified by an ultracentrifugation method.

It was clearly demonstrated from the above-described result that the impurity removal method using light basic magnesium carbonate has little effect on an infectivity titer of AAV.

### Example 14: Impurity removal from lentivirus culture fluid (1) Preparation of lentivirus culture supernatant

Cultured Lenti-X293T cell (manufactured by Takara Bio) was seeded in a concentration of 1.5 to 1.8 × 10⁶ cells/well on a 6 well plate coated using collagen for cell culture ("AteloCell" manufactured by KOKEN) and incubated at 37°C for 24 hours. The cell was transfected with transfer plasmid CS VI-CMV-Venus (reference document: WO 2018/088519) and packaging plasmid ("3rd Generation pLenti Combo Mix" manufactured by Applied Biological) using lipofectamine and incubated at 37°C for 6 hours. Then, the culture medium was exchanged, and the cell was cultivated at 37°C for 72 hours to produce lentivirus. The culture supernatant was recovered and filtrated using a syringe filter having a pore diameter of 0.8 µm to remove the cell and obtain the culture supernatant containing lentivirus.

### (2) Impurity removal from lentivirus culture fluid

To 5 mL of the culture supernatant, 50 mg or 500 mg of basic magnesium carbonate was added. The mixture was shaken at room temperature for 1 minute or 1 hour. Each cell dissolution liquid without an additive was shaken in the same condition as control. The supernatant was obtained by centrifugation, and the lentivirus amount in the obtained supernatant was measured using an easy lentivirus amount measurement reagent ("Lenti-X GoStix Plus" manufactured by Takara Bio). In addition, the protein concentration was measured by the same method as Example 1. Furthermore, the obtained supernatant was centrifuged at 18,000 rpm and at 4°C for 2 hours to precipitate lentivirus. The lentivirus was suspended in 1/10 amount of PBS to the centrifuged obtained supernatant after the supernatant was removed. The suspension was referred to as the virus concentration liquid, and the protein concentration was measured. The result is shown in Table 16.

**Table 16**

| No. | Treatment time | Additive amount of water-insoluble inorganic compound | Protein concentration (mg/mL) | |
|---|---|---|---|---|
| | | | Recovered liquid | Virus concentrate |
| 1 | 1 min | - | 1.2 | 0.2 |
| 2 | | 1% | 0.9 | 0.2 |
| 3 | | 10% | 0.3 | 0 |
| 4 | 60 min | - | 1.1 | 0.4 |
| 5 | | 1% | 0.9 | 0.2 |
| 6 | | 10% | 0.4 | 0 |

The amount of lentivirus in each recovered liquid was measured using an easy lentivirus amount measurement reagent; as a result, it was confirmed that lentivirus could be recovered from any recovered liquids, since there were the bands of lentivirus. Since the difference between the band intensities could not be visually recognized, it was judged that similar amounts of lentivirus could be recovered. In addition, the protein concentrations in the recovered liquid and the virus concentration liquid were measured; as a result, the protein concentration in the sample to which basic magnesium carbonate was added was low as the result shown in Table 16.

It was clearly found from the above result that the impurity removal method using basic magnesium carbonate according to the present invention can be utilized for an envelope virus such as lentivirus.

## Claims

1. A method for purifying a virus or a virus-like particle,
comprising the step of contacting a liquid comprising the virus or the virus-like particle with a water-insoluble inorganic compound,
wherein the water-insoluble inorganic compound comprises one or more elements selected from magnesium, calcium and aluminum.

2. A method for producing a virus or a virus-like particle,
comprising the step of purifying the virus or the virus-like particle by contacting a liquid comprising the virus or the virus-like particle with a water-insoluble inorganic compound,
wherein the water-insoluble inorganic compound comprises one or more elements selected from magnesium, calcium and aluminum.

3. The method according to claim 1 or 2, wherein the water-insoluble inorganic compound is one or more selected from magnesium carbonate, magnesium hydroxide, magnesium oxide, calcium sulfate and aluminum oxide.

4. The method according to any one of claims 1 to 3, further comprising the step of contacting the liquid comprising the virus or the virus-like particle with an activated carbon.

5. The method according to any one of claims 1 to 4, further comprising the step of purifying the virus or the virus-like particle by chromatography.

6. The method according to claim 5, wherein the chromatography is affinity chromatography.

7. The method according to any one of claims 1 to 6, wherein the virus or the virus-like particle is an adeno-associated virus or a virus-like particle derived from an adeno-associated virus.

8. The method according to any one of claims 1 to 7, wherein a protein and/or a nucleic acid other than the virus or the virus-like particle is adsorbed on the water-insoluble inorganic compound.

9. The method according to any one of claims 1 to 8, wherein the liquid is an animal cell culture fluid or a processed material thereof.


SEQUENCE LISTING

<110>  KANEKA CORPORATION

<120>  Method for Purifying Virus or Virus-like Particle

<130> B190146

<140> JP2019-125243
<141> 2019-07-04

<160>  2

<210>  1
<211>  15
<212>  DNA
<213>  Artificial Sequence

<220>
<223>  primer

<400>  1

gaggcgggcg gatca       15


<210>  2
<211>  27
<212>  DNA
<213>  Artificial Sequence

<220>
<223>  primer

<400>  2

cccggctaat ttttgtattt ttagtag       27